(19) Europäisches Patentamt
European Patent Office
Office européen des brevets

(11) EP 2 198 838 B1

(12) # FASCICULE DE BREVET EUROPEEN

(45) Date de publication et mention de la délivrance du brevet:
**05.09.2018 Bulletin 2018/36**

(51) Int Cl.:
*A61K 8/31* (2006.01) *A61K 8/41* (2006.01)
*A61K 8/86* (2006.01) *A61Q 5/08* (2006.01)
*A61Q 5/06* (2006.01) *A61Q 5/10* (2006.01)

(21) Numéro de dépôt: **09179844.7**

(22) Date de dépôt: **18.12.2009**

(54) **Procédé et dispositif d'éclaircissement ou de coloration directe éclaircissante ou d'oxydation des fibres kératiniques en présence d'une composition aqueuse riche en corps gras**

Verfahren und Kit zum Aufhellen oder direkten oder oxidativen Färben keratinischer Fasern mit einer wässrigen Zusammensetzung reich an Fettkörpern

Process and kit for lightening or direct or oxidative dyeing keratinic fibers with an aqueous composition rich in fatty compounds

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **19.12.2008 FR 0858880**
**19.12.2008 FR 0858888**

(43) Date de publication de la demande:
**23.06.2010 Bulletin 2010/25**

(73) Titulaire: **L'Oréal**
**75008 Paris (FR)**

(72) Inventeur: **Hercouet, Leïla**
**93360, Neuilly Plaisance (FR)**

(74) Mandataire: **Dossmann, Gérard**
**Casalonga & Partners**
**Bayerstrasse 71-73**
**80335 München (DE)**

(56) Documents cités:
**CA-A1- 2 573 567** **DE-A1- 3 814 356**
**DE-A1- 10 028 723** **DE-A1- 10 056 266**
**DE-A1- 10 148 671** **DE-A1- 19 712 980**
**FR-A- 2 910 309**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention a pour objet un procédé de coloration des fibres kératiniques, en particulier des fibres kératiniques humaines telles que les cheveux qui comprend la mise en oeuvre d'une composition cosmétique aqueuse (A) comprenant un ou plusieurs corps gras et un ou plusieurs tensioactifs, d'une composition cosmétique (B) comprenant un ou plusieurs agents alcalins particuliers et en outre un ou plusieurs colorants, et d'une composition cosmétique (C) comprenant un ou plusieurs oxydants.

La présente invention a trait au domaine de la coloration des fibres kératiniques et plus particulièrement au domaine de la coloration capillaire.

Depuis longtemps, de nombreuses personnes cherchent à modifier la couleur de leurs cheveux et en particulier à masquer leurs cheveux blancs. Pour ce faire, il existe essentiellement deux types de coloration qui ont été développées.

Le premier type de coloration est la coloration dite permanente ou d'oxydation qui met en oeuvre des compositions tinctoriales contenant des précurseurs de colorant d'oxydation, appelés généralement bases d'oxydation. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques tels que des composés indoliques. La variété des molécules mises en jeu au niveau des bases d'oxydation et des coupleurs permet l'obtention d'une riche palette de couleurs.

Le deuxième type de coloration est la coloration dite semi-permanente ou coloration directe qui consiste à appliquer sur les fibres kératiniques des colorants directs qui sont des molécules colorées et colorantes, ayant une affinité pour les fibres, à laisser pauser pour permettre aux molécules de pénétrer, par diffusion à l'intérieur de la fibre, puis à les rincer.

Afin de réaliser ces colorations, les colorants directs généralement employés sont choisis parmi les colorants directs nitrés benzéniques, anthraquinoniques, nitropyridiniques, azoïques, xanthéniques, acridiniques, aziniques ou triarylméthaniques.

Ce type de procédé ne nécessite pas l'emploi d'un agent oxydant pour développer la coloration. Cependant, il n'est pas exclu d'en mettre en oeuvre afin d'obtenir un effet d'éclaircissement avec la coloration. On parle alors d'une coloration directe ou semi-permanente en conditions éclaircissantes.

Les procédés de coloration permanente ou encore semi-permanente en conditions éclaircissantes, consistent donc à employer avec la composition tinctoriale, une composition aqueuse comprenant au moins un agent oxydant, en condition de pH alcalin dans la grande majorité des cas. Cet agent oxydant a pour rôle, au moins en partie, de dégrader la mélanine des cheveux, ce qui, en fonction de la nature de l'agent oxydant présent, conduit à un éclaircissement plus ou moins prononcé des fibres. Ainsi, pour un éclaircissement relativement faible, l'agent oxydant est généralement le peroxyde d'hydrogène. Lorsqu'un éclaircissement plus important est recherché, on met habituellement en oeuvre des sels peroxygénés, comme des persulfates par exemple, en présence de peroxyde d'hydrogène.

Les procédés d'éclaircissement des fibres kératiniques humaines consistent à mettre en oeuvre une composition aqueuse comprenant au moins un agent oxydant dans des conditions de pH alcalin dans la grande majorité des cas. Cet agent oxydant a pour rôle de dégrader la mélanine des cheveux, ce qui, en fonction de la nature de l'agent oxydant présent, conduit à un éclaircissement plus ou moins prononcé des fibres. Ainsi, pour un éclaircissement relativement faible, l'agent oxydant est généralement le peroxyde d'hydrogène. Lorsqu'un éclaircissement plus important est recherché, on met habituellement en oeuvre des sels peroxygénés, comme des persulfates par exemple, en présence de peroxyde d'hydrogène.

L'une des difficultés rencontrées lors de la mise en oeuvre des procédés d'éclaircissement et de coloration de l'art antérieur vient du fait que ces procédés sont mis en oeuvre dans des conditions alcalines et que l'agent alcalin le plus couramment utilisé est l'ammoniaque. L'emploi de l'ammoniaque est particulièrement avantageux dans ce type de procédés. En effet, il permet d'ajuster le pH de la composition à un pH alcalin pour permettre l'activation de l'agent oxydant. Mais cet agent alcalin provoque également un gonflement de la fibre kératinique, avec un soulèvement des écailles, ce qui favorise la pénétration de l'oxydant, ainsi que s'ils sont présents des colorants, essentiellement les colorants d'oxydation, à l'intérieur de la fibre, et donc augmente l'efficacité de la réaction de coloration.

Or cet agent alcalinisant est très volatil, ce qui occasionne des désagréments à l'utilisateur du fait de l'odeur caractéristique forte, plutôt incommodante de l'ammoniac qui se dégage durant le procédé.

De plus, la quantité d'ammoniac dégagée nécessite l'emploi de teneurs plus importantes que nécessaires pour compenser cette perte. Cela n'est pas sans conséquence pour l'utilisateur qui reste non seulement incommodé par l'odeur mais qui peut également être confronté à des risques plus importants d'intolérance, comme par exemple une irritation du cuir chevelu se traduisant notamment par des picotements.

Quant à l'option de purement et simplement remplacer en totalité ou en partie l'ammoniaque par un ou plusieurs

autres agents alcalinisants classiques, celle-ci ne conduit pas à des compositions aussi efficaces que celles à base d'ammoniaque, notamment parce que ces agents alcalinisants ne conduisent pas un éclaircissement suffisant des fibres pigmentées en présence de l'agent oxydant.

CA 2 573 567 A1 (PROD VERNICO LTEE [CA]) 16 mars 2006 décrit dans les exemples un procédé pour la coloration ou la décoloration des cheveux, comprenant le mélange extemporané de trois compositions:

- composition A (exemple 1) contient 24% de corps gras et un tensioactif non-ionique,
- composition B (exemple 2) contient la monoéthanolamine comme agent alcalinisant,
- et composition C (exemple 3) contient du peroxyde d'hydrogène.

US 5 817 155 explique qu'en ajoutant de l'alcool isostéarylique à une composition colorante un effet colorant supérieur est obtenu.

L'un des objectifs de la présente invention est de proposer des procédés de coloration des fibres kératiniques humaines mis en oeuvre en présence d'un agent oxydant qui n'ont pas les inconvénients des procédés existants, inconvénients dus à la présence de teneurs importantes en ammoniaque, mais qui restent au moins aussi efficaces. les procédés de coloration doivent être efficaces sur le plan de la puissance de la coloration obtenue, ainsi que sur celui de la chromaticité, de l'homogénéité de la coloration le long de la fibre.

Ces buts et d'autres sont atteints par la présente invention qui a donc pour objet un procédé de coloration des fibres kératiniques, en particulier des fibres kératiniques humaines telles que les cheveux dans lequel on applique sur lesdites fibres :

(a) une composition cosmétique aqueuse (A) comprenant un ou plusieurs corps gras et un ou plusieurs tensioactifs ;
(b) une composition cosmétique (B) comprenant un ou plusieurs agents alcalins,
(c) une composition (C) comprenant un ou plusieurs agents oxydants,

la quantité des corps gras de la composition (A) étant supérieure à 20% en poids par rapport au poids total de la composition (A) la composition cosmétique (B) comprend en outre un ou plusieurs colorants d'oxydation et/ou un ou plusieurs colorants directs.

Le procédé de coloration selon l'invention conduit à des colorations puissantes et peu sélectives, c'est-à-dire des colorations qui sont homogènes le long de la fibre.

Par ailleurs, les procédés selon l'invention permettent de conduire à des compositions qui ne dégagent pas une odeur agressive lors de leur application sur les cheveux ou lors de leur préparation.

D'autres caractéristiques et avantages de l'invention apparaîtront plus clairement à la lecture de la description et des exemples qui suivent.

Dans ce qui va suivre, et à moins d'une autre indication, les bornes d'un domaine de valeurs sont comprises dans ce domaine.

Les fibres kératiniques humaines traitées par le procédé selon l'invention sont de préférence les cheveux.

Comme indiqué auparavant, le procédé selon l'invention est mis en oeuvre en présence d'une composition cosmétique aqueuse comprenant un ou plusieurs corps gras (A).

Par composition aqueuse, on entend une composition comprenant plus de 5 % en poids d'eau, de préférence plus de 10 % en poids d'eau, et de manière encore plus avantageuse plus de 20 % en poids d'eau. Ainsi que cela a été mentionné, la quantité des corps gras de la composition cosmétique aqueuse (A) est supérieure à 20% en poids par rapport au poids total de la composition (A).

Par corps gras, on entend, un composé organique insoluble dans l'eau à température ordinaire (25°C) et à pression atmosphérique (760 mm de Hg) c'est-à-dire de solubilité inférieure à 5% et de préférence à 1% encore plus préférentiellement à 0,1%. Ils présentent dans leur structure au moins un enchaînement d'au moins deux groupements siloxanes ou une chaîne hydrocarbonée comportant au moins 6 atomes de carbone. En outre, les corps gras sont généralement solubles dans des solvants organiques dans les mêmes conditions de température et de pression, comme par exemple le chloroforme, l'éthanol, le benzène, l'huile de vaseline ou le décaméthylcyclopentasiloxane.

Les corps gras sont notamment choisis parmi l'huile de vaseline, les polydécènes liquides ou leurs mélanges.

Il est rappelé qu'au sens de l'invention, les alcools, esters et acides gras présentent plus particulièrement un ou plusieurs groupements hydrocarbonés, linéaires ou ramifiés, saturés ou insaturés, comprenant 6 à 30 atomes de carbone, éventuellement substitués, en particulier par un ou plusieurs groupements hydroxyle (en particulier 1 à 4). S'ils sont insaturés, ces composés peuvent comprendre une à trois double-liaisons carbone-carbone, conjuguées ou non.

Les esters d'acide gras et/ou d'alcool gras, avantageusement différents des triglycérides utilisables dans la composition (A) sont les esters de mono ou polyacides aliphatiques saturés ou insaturés, linéaires ou ramifiés en $C_1$-$C_{26}$

et de mono ou polyalcools aliphatiques saturés ou insaturés, linéaires ou ramifiés en $C_1$-$C_{26}$, le nombre total de carbone des esters étant plus particulièrement supérieur ou égal à 10.

Parmi les monoesters , on peut citer le béhénate de dihydroabiétyle ; le béhénate d'octyldodécyle ; le béhénate d'isocétyle ; le lactate de cétyle ; le lactate d'alkyle en $C_{12}$-$C_{15}$ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, de myristyle, de stéaryle, le stéarate d'hexyle, le stéarate de butyle, le stéarate d'isobutyle ; le malate de dioctyle, le laurate d'hexyle, le laurate de 2-hexyldécyle.

Toujours dans le cadre de cette variante, on peut également utiliser les esters d'acides di ou tricarboxyliques en $C_4$-$C_{22}$ et d'alcools en $C_1$-$C_{22}$ et les esters d'acides mono di ou tricarboxyliques et d'alcools di, tri, tétra ou pentahydroxy en $C_2$-$C_{26}$.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol, l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle, le dioctanoate de propylène glycol ; le diheptanoate de néopentyl glycol ; le diisanonate de diéthylène glycol ; et les distéarates de polyéthylène glycol.

Parmi les esters cités ci-dessus, on préfère utiliser les palmitates d'éthyle, d'isopropyle, de myristyle, de cétyle, de stéaryle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

La composition (A) peut également comprendre, à titre d'ester gras, des esters et di-esters de sucres d'acides gras en $C_6$-$C_{30}$, de préférence en $C_{12}$-$C_{22}$. Il est rappelé que l'on entend par « sucre », des composés hydrocarbonés oxygénés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres convenables, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras peuvent être choisis notamment dans le groupe comprenant les esters ou mélanges d'esters de sucres décrits auparavant et d'acides gras en $C_6$-$C_{30}$, de préférence en $C_{12}$-$C_{22}$, linéaires ou ramifiés, saturés ou insaturés. S'ils sont insaturés, ces composés peuvent comprendre une à trois double-liaisons carbone-carbone, conjuguées ou non.

Les esters selon cette variante peuvent être également choisis parmi les mono-, di-, tri- et tétra-esters, les polyesters et leurs mélanges.

Ces esters peuvent être par exemple des oléate, laurate, palmitate, myristate, béhénate, cocoate, stéarate, linoléate, linolénate, caprate, arachidonates, ou leurs mélanges comme notamment les esters mixtes oléo-palmitate, oléo-stéarate, palmito-stéarate.

Plus particulièrement, on utilise les mono- et di- esters et notamment les mono- ou di- oléate, stéarate, béhénate, oléopalmitate, linoléate, linolénate, oléostéarate de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :

- les produits vendus sous les dénominations F160, F140, F110, F90, F70, SL40 par la société Crodesta, désignant respectivement les palmito-stéarates de sucrose formés de 73 % de monoester et 27 % de di- et tri-ester, de 61 % de monoester et 39 % de di-, tri-, et tétra-ester, de 52 % de monoester et 48 % de di-, tri-, et tétra-ester, de 45 % de monoester et 55 % de di-, tri-, et tétra-ester, de 39 % de monoester et 61 % de di-, tri-, et tétra-ester, et le mono-laurate de sucrose;
- les produits vendus sous la dénomination Ryoto Sugar Esters par exemple référencés B370 et correspondant au béhénate de saccharose formé de 20 % de monoester et 80 % de di-triester-polyester;
- le mono-di-palmito-stéarate de sucrose commercialisé par la société Goldschmidt sous la dénomination Tegosoft®

PSE.

De préférence, les corps gras ne comprennent pas de motifs oxyalkylénés en $C_2$-$C_3$, ni de motifs glycérolés.
Plus particulièrement, les corps gras sont choisis parmi les composés liquides ou pâteux à température ambiante et à pression atmosphérique.
De préférence, le corps gras est un composé liquide à la température de 25°C et à la pression atmosphérique.
Les corps gras sont de préférence choisis parmi les esters d'acide gras et/ou d'alcool gras,
le ou les corps gras est ou sont choisis parmi l'huile de vaseline, les polydécènes, les esters d'acides gras et/ou d'alcool gras, liquides ou leurs mélanges,
**[0046]** De préférence, le ou les corps gras de la composition selon l'invention sont non siliconés.
La composition aqueuse (A) selon l'invention comprend le corps gras à la concentration de 25 à 80 %, encore plus préférentiellement de 25 à 65 %, mieux de 30 à 55 % du poids total de la composition.
La composition cosmétique aqueuse (A) comprend également un ou plusieurs tensioactifs.
De préférence, le ou les tensioactifs sont choisis parmi les tensioactifs non ioniques ou parmi les tensioactifs anioniques.
Les tensioactifs anioniques sont plus spécialement choisis parmi les sels (en particulier sels de métaux alcalins, notamment de sodium, sels d'ammonium, sels d'amines tels que les sels d'aminoalcools ou sels de métaux alcalino-terreux comme le magnésium) des composés suivants :

- les alkylsulfates, les alkyléthersulfates, alkylamidoéthersulfates, alkylaryl-polyéthersulfates, monoglycérides sulfates ;
- les alkylsulfonates, alkylamidesulfonates, alkylarylsulfonates, $\alpha$-oléfine-sulfonates, paraffine-sulfonates ;
- les alkylphosphates, les alkylétherphosphates;
- les alkylsulfosuccinates, les alkyléthersulfosuccinates, les alkylamide-sulfosuccinates; les alkylsulfosuccinamates ;
- les alkylsulfoacétates ;
- les acylsarcosinates ; les acyliséthionates et les N-acyltaurates ;
- les sels d'acides gras tels que les acides oléique, ricinoléique, palmitique, stéarique, les acides d'huile de coprah ou d'huile de coprah hydrogénée ;
- les sels d'acides d'alkyl D galactoside uroniques ;
- les acyl-lactylates ;
- les sels des acides alkyléther carboxyliques polyoxyalkylénés, des acides alkylaryléther carboxyliques polyoxyalkylénés, des acides alkylamidoéther carboxyliques polyoxyalkylénés, en particulier ceux comportant de 2 à 50 groupements oxyde d'éthylène ;
- et leurs mélanges.

Il est à noter que le radical alkyle ou acyle de ces différents composés comporte avantageusement de 6 à 24 atomes de carbone, et de préférence de 8 à 24 atomes de carbone, et le radical aryle désignant de préférence un groupement phényle ou benzyle.
Les tensioactifs non ioniques sont plus particulièrement choisis parmi les tensioactifs non ioniques mono ou poly- oxyalkylénés, mono- ou poly- glycérolés. Les motifs oxyalkylénés sont plus particulièrement des motifs oxyéthylénés, oxypropylénés, ou leur combinaison, de préférence oxyéthylénés.
A titre d'exemples de tensioactifs non ioniques oxyalkylénés, on peut citer :

- les alkyl($C_8$-$C_{24}$)phénols oxyalkylénés,
- les alcools en $C_8$-$C_{30}$, saturés ou non, linéaires ou ramifiés, oxyalkylénés,
- les amides, en $C_8$-$C_{30}$, saturés ou non, linéaires ou ramifiés, oxyalkylénés,

- les esters d'acides en $C_8$-$C_{30}$, saturés ou non, linéaires ou ramifiés, et de polyéthylèneglycols,
- les esters d'acides en $C_8$-$C_{30}$, saturés ou non, linéaires ou ramifiés, et de sorbitol polyoxyéthylénés,
- les huiles végétales oxyéthylénées, saturées ou non,
- les condensats d'oxyde d'éthylène et/ou d'oxyde de propylène, entre autres, seuls ou en mélanges.

Les tensioactifs présentant un nombre de moles d'oxyde d'éthylène et/ou de propylène compris entre 1 et 100, de préférence entre 2 et 50. De manière avantageuse, les tensioactifs non ioniques ne comprennent pas de motifs oxypropylénés.

Conformément à un mode de réalisation préféré de l'invention, les tensioactifs non ioniques oxyalkylénés sont choisis parmi les alcools en $C_8$-$C_{30}$, oxyéthylénés, les esters d'acides en $C_8$-$C_{30}$, saturés ou non, linéaires ou ramifiés, et de sorbitol polyoxyéthylénés.

A titre d'exemple de tensioactifs non ioniques mono- ou poly-glycérolés, on utilise de préférence les alcools en $C_8$-$C_{40}$, mono- ou poly- glycérolés.

En particulier, les alcools en $C_8$-$C_{40}$ mono- ou poly- glycérolés correspondent à la formule suivante :

$$RO\text{-}[CH_2\text{-}CH(CH_2OH)\text{-}O]_m\text{-}H$$

dans laquelle R représente un radical alkyle ou alcényle, linéaire ou ramifié, en $C_8$-$C_{40}$, de préférence en $C_8$-$C_{30}$, et m représente un nombre allant de 1 à 30 et de préférence de 1 à 10.

A titre d'exemple de composés convenables dans le cadre de l'invention, on peut citer, l'alcool laurique à 4 moles de glycérol (nom INCI : POLYGLYCERYL-4 LAURYL ETHER), l'alcool laurique à 1,5 moles de glycérol, l'alcool oléique à 4 moles de glycérol (nom INCI : POLYGLYCERYL-4 OLEYL ETHER), l'alcool oléique à 2 moles de glycérol (Nom INCI : POLYGLYCERYL-2 OLEYL ETHER), l'alcool cétéarylique à 2 moles de glycérol, l'alcool cétéarylique à 6 moles de glycérol, l'alcool oléocétylique à 6 moles de glycérol, et l'octadécanol à 6 moles de glycérol.

L'alcool peut représenter un mélange d'alcools au même titre que la valeur de m représente une valeur statistique, ce qui signifie que dans un produit commercial peuvent coexister plusieurs espèces d'alcools gras polyglycérolés sous forme d'un mélange.

Parmi les alcools mono- ou poly-glycérolés, on préfère plus particulièrement utiliser l'alcool en $C_8/C_{10}$ à une mole de glycérol, l'alcool en $C_{10}/C_{12}$ à 1 mole de glycérol et l'alcool en $C_{12}$ à 1,5 mole de glycérol:

De préférence, le tensioactif présent dans la composition de l'invention est un tensioactif non ionique.

La teneur en tensioactifs dans la composition de l'invention représente plus particulièrement de 0,1 à 50 % en poids, de préférence de 0,5 à 30 % en poids par rapport au poids de la composition.

La composition cosmétique aqueuse (A) peut également renfermer divers adjuvants utilisés classiquement dans les compositions pour l'éclaircissement ou la coloration des cheveux, tels que des polymères anioniques, cationiques, non ioniques, amphotères, zwitterioniques ou leurs mélanges ; des agents épaississants minéraux, et en particulier des charges telles que des argiles, le talc ; des agents épaississants organiques, avec en particulier les épaississants associatifs polymères anioniques, cationiques, non ioniques et amphotères ; des agents antioxydants ; des agents de pénétration ; des agents séquestrants ; des parfums ; des agents dispersants ; des agents filmogènes ; des céramides ; des agents conservateurs ; des agents opacifiants.

Les adjuvants ci dessus sont en général présents en quantité comprise pour chacun d'eux entre 0,01 et 20 % en poids par rapport au poids de la composition (A).

Selon une variante avantageuse de l'invention, la composition aqueuse (A) comprend un ou plusieurs agents épaississants minéraux choisis parmi les argiles organophiles, les silices pyrogénées, ou leurs mélanges.

L'argile organophile peut être choisie parmi la montmorrilonite, la bentonite, l'hectorite, l'attapulgite, la sépiolite, et leurs mélanges. L'argile est de préférence une bentonite ou une hectorite.

Ces argiles peuvent être modifiées avec un composé chimique choisi parmi les amines quaternaires, les amines tertiaires, les acétates aminés, les imidazolines, les savons aminés, les sulfates gras, les alkyl aryl sulfonates, les oxides amines, et leurs mélanges.

Comme argiles organophiles, on peut citer les quaternium-18 bentonites telles que celles vendues sous les dénominations Bentone 3, Bentone 38, Bentone 38V par la société Rhéox, Tixogel VP par la société United catalyst, Claytone 34, Claytone 40, Claytone XL par la société Southern Clay; les stéaralkonium bentonites telles que celles vendues sous les dénominations Bentone 27 par la société Rheox, Tixogel LG par la société United Catalyst, Claytone AF, Claytone APA par la société Southern Clay ; les quaternium-18/benzalkonium bentonite telles que celles vendues sous les dénominations Claytone HT, Claytone PS par la société Southern Clay, les Quaternium-18 Hectorites telles que celles vendues sous les dénominations Bentone Gel DOA, Bentone Gel ECO5, Bentone Gel EUG, Bentone Gel

IPP, Bentone Gel ISD, Bentone Gel SS71, Bentone Gel VS8, Bentone Gel VS38 par la société Rhéox et Simagel M, Simagel SI 345 par la société Biophil.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leur surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot.

Il est possible de modifier chimiquement la surface de la silice par réaction chimique en vue de diminuer le nombre de groupes silanol. On peut notamment substituer des groupes silanol par des groupements hydrophobes : on obtient alors une silice hydrophobe.

Les groupements hydrophobes peuvent être :

- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénommées "Silica diméthyl silylate" selon le CTFA (6ème édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

La silice pyrogénée présente de préférence une taille de particules pouvant être nanométrique à micrométrique, par exemple allant d'environ de 5 à 200 nm.

De préférence, la composition comprend une hectorite, une bentonite organomodifiée ou une silice pyrogénée éventuellement modifiée.

Lorsqu'il est présent, l'agent épaississant minéral représente de 1 à 30 % en poids par rapport au poids de la composition.

La composition peut également comprendre un ou plusieurs agents épaississants organiques.

Ces agents épaississants peuvent être choisis parmi les amides d'acides gras (diéthanol- ou monoéthanolamide de coprah, monoéthanolamide d'acide alkyl éther carboxylique oxyéthyléné), les épaississants polymériques tels que les épaississants cellulosiques (hydroxyéthycellulose, hydroxypropylcellulose, carboxyméthyl-cellulose), la gomme de guar et ses dérivés (hydroxypropylguar), les gommes d'origine microbienne (gomme de xanthane, gomme de scléroglucane), les homopolymères réticulés d'acide acrylique ou d'acide acrylamidopropanesulfonique et les polymères associatifs (polymères comprenant des zones hydrophiles, et des zones hydrophobes à chaîne grasse (alkyle, alcényle comprenant au moins 10 atomes de carbone) capables, dans un milieu aqueux, de s'associer réversiblement entre eux ou avec d'autres molécules.).

Selon un mode de réalisation particulier, l'épaississant organique est choisi parmi les épaississants cellulosiques (hydroxyéthycellulose, hydroxypropylcellulose, carboxyméthyl-cellulose), la gomme de guar et ses dérivés (hydroxypropylguar), les gommes d'origine microbienne (gomme de xanthane, gomme de scléroglucane), les homopolymères réticulés d'acide acrylique ou d'acide acrylamidopropanesulfonique, et de préférence parmi les épaississants cellulosiques avec en particulier l'hydroxyéthycellulose.

La teneur en agent(s) épaississant(s) organique(s), s'ils sont présents, varie habituellement de 0,01 % à 20 % en poids, par rapport au poids de la composition, de préférence de 0,1 à 5 % en poids.

Avantageusement, la composition (A) se présente sous la forme d'un gel ou d'une crème
le procédé selon l'invention est mis en oeuvre en présence d'une composition cosmétique (B) comprenant un ou plusieurs colorants d'oxydation, un ou plusieurs colorants directs, ou leurs mélanges.

Les colorants d'oxydation sont en général choisis parmi les bases d'oxydation éventuellement combinée(s) à un ou plusieurs coupleurs.

A titre d'exemple, les bases d'oxydation sont choisies parmi les paraphénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4-5-diamino 1-(β-méthoxyéthyl)pyrazole.

De préférence, on utilisera un 4,5-diaminopyrazole et encore plus préférentiellement le 4,5-diamino-1-(β-hydroxyéthyl)-pyrazole et/ou l'un des ses sels.

A titre de dérivés pyrazoliques, on peut également citer les diamino N,N-dihydropyrazolopyrazolones et notamment celles décrites dans la demande FR-A-2 886 136 telles que les composés suivants et leurs sels d'addition : 2,3-diamino-6,7-dihydro-1H,5H-pyrazolo[1,2-a]pyrazol-1-one, 2-amino-3-éthylamino-6,7-dihydro-1H,5H-pyrazolo[1,2-a]pyrazol-1-one, 2-amino-3-isopropylamino-6,7-dihydro-1H,5H-pyrazolo[1,2-a]pyrazol-1-one, 2-amino-3-(pyrrolidin-1-yl)-6,7-dihydro-1H,5H-pyrazolo[1,2-a]pyrazol-1-one, 4,5-diamino-1,2-diméthyl-1,2-dihydro-pyrazol-3-one, 4,5-diamino-1,2-diéthyl-1,2-dihydro-pyrazol-3-one, 4,5-diamino-1,2-di-(2-hydroxyéthyl)-1 ,2-dihydro-pyrazol-3-one, 2-amino-3-(2-hydroxyéthyl)amino-6,7-dihydro-1H,5H-pyrazolo[1,2-a]pyrazol-1-one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-amino-1,2-diéthyl-5-(pyrrolidin-1-yl)-1,2-dihydro-pyrazol-3-one, 4-amino-5-(3-diméthylamino-pyrrolidin-1-yl)-1,2-diéthyl-1,2-dihydro-pyrazol-3-one, 2,3-diamino-6-hydroxy-6,7-dihydro-1H,5H-pyrazolo[1,2-a]pyrazol-1-one.

On préférera utiliser la 2,3-diamino-6,7-dihydro-1H,5H-pyrazolo[1,2-a]pyrazol-1-one et/ou un de ses sels.

A titre de bases hétérocycliques, on utilisera préférentiellement le 4,5-diamino-1-(β-hydroxyéthyl)pyrazole et/ou la 2,3-diamino-6,7-dihydro-1H,5H-pyrazolo[1,2-a]pyrazol-1-one et/ou un de leurs sels.

La composition cosmétique (B) selon l'invention peut éventuellement comprendre un ou plusieurs coupleurs choisis avantageusement parmi ceux conventionnellement utilisés pour la teinture des fibres kératiniques.

Parmi ces coupleurs, on peut notamment citer les méta-phénylènediamines, les méta-aminophénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(13-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, leurs sels d'addition avec un acide, et leurs mélanges.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

La ou les bases d'oxydation représentent chacune avantageusement de 0,0001 à 10 % en poids par rapport au poids total de la composition, et de préférence de 0,005 à 5 % en poids par rapport au poids total de la composition.

La teneur en coupleur(s), s'il(s) est(sont) présent(s), représente chacun avantageusement de 0,0001 à 10 % en poids par rapport au poids total de la composition, et de préférence de 0,005 à 5 % en poids par rapport au poids total de la composition cosmétique (B).

En ce qui concerne les colorants directs, ces derniers sont plus particulièrement choisis parmi les espèces ioniques ou non ioniques, de préférence cationiques ou non ioniques.

A titre d'exemples de colorants directs convenables, on peut citer les colorants directs azoïques ; méthiniques ; carbonyles ; aziniques ; nitrés (hétéro)aryle ; tri-(hétéro)aryle méthanes ; les porphyrines ; les phtalocyanines et les colorants directs naturels, seuls ou en mélanges.

Plus particulièrement, les colorants azoïques comprennent une fonction -N=N- dont les deux atomes d'azote ne sont pas simultanément engagés dans un cycle. Il n'est toutefois pas exclu que l'un des deux atomes d'azote de l'enchaînement -N=N- soit engagé dans un cycle.

Les colorants de la famille des méthines sont plus particulièrement des composés comprenant au moins un enchaînement choisi parmi >C=C< et -N=C< dont les deux atomes ne sont pas simultanément engagés dans un cycle. Il est toutefois précisé que l'un des atomes d'azote ou de carbone des enchaînements peut être engagé dans un cycle. Plus particulièrement, les colorants de cette famille sont issus de composés de type méthine, azométhine, mono- et di-arylméthane, indoamines (ou diphénylamines), indophénols, indoanilines, carbocyanines, azacabocyanines et leurs isomères, diazacarbocyanines et leurs isomères, tétraazacarbocyanines, hémicyanines

Concernant les colorants de la famille des carbonyles, on peut citer par exemple les colorants choisis parmi les acridone, benzoquinone, anthraquinone, naphtoquinone, benzanthrone, anthranthrone, pyranthrone, pyrazolanthrone, pyrimidinoanthrone, flavanthrone, idanthrone, flavone, (iso)violanthrone, isoindolinone, benzimidazolone, isoquinolinone, anthrapyridone, pyrazoloquinazolone, périnone, quinacridone, quinophthalone, indigoïde, thioindigo, naphtalimide, anthrapyrimidine, dicétopyrrolopyrrole, coumarine.

Concernant les colorants de la famille des azines cycliques, on peut citer notamment les azine, xanthène, thioxanthène, fluorindine, acridine, (di)oxazine, (di)thiazine, pyronine.

Les colorants nitrés (hétéro)aromatiques sont plus particulièrement des colorants directs nitrés benzéniques ou nitrés pyridiniques.

Concernant les colorants de type porphyrines ou phtalocyanines, on peut mettre en oeuvre des composés cationiques ou non, comprenant éventuellement un ou plusieurs métaux ou ions métalliques, comme par exemple des métaux alcalins et alcalino-terreux, le zinc et le silicium.

A titre d'exemple de colorants directs particulièrement convenables, on peut citer les colorants nitrés de la série benzénique ; les colorants directs azoïques ; azométhiniques ; méthiniques ; les azacarbocyanines comme les tétraazacarbocyanines (tétraazapentaméthines) ; les colorants directs quinoniques et en particulier anthraquinoniques, naphtoquinoniques ou benzoquinoniques ; les colorants directs aziniques ; xanthéniques ; triarylméthaniques ; indoaminiques ; indigoïdes ; phtalocyanines, porphyrines et les colorants directs naturels, seuls ou en mélanges.

Ces colorants peuvent être des colorants monochromophoriques (c'est-à-dire ne comprenant qu'un seul colorant) ou polychromophoriques, de préférence di- ou tri- chromophoriques ; les chromophores pouvant être identiques ou non, de la même famille chimique ou non. A noter que qu'un colorant polychromophorique comprend plusieurs radicaux issus chacun d'une molécule absorbant dans le domaine visible entre 400 et 800 nm. De plus cette absorbance du colorant ne nécessite ni oxydation préalable de celui-ci, ni association avec d'autre(s) espèce(s) chimique(s).

Dans le cas de colorants polychromophoriques, les chromophores sont reliés entre eux au moyen d'au moins un bras de liaison qui peut être cationique ou non.

De préférence, le bras de liaison est une chaîne alkyle en $C_1$-$C_{20}$, linéaire, ramifiée ou cyclique, éventuellement interrompue par au moins un hétéroatome (tel que l'azote, l'oxygène) et/ou par au moins un groupe en comprenant (CO, $SO_2$), éventuellement interrompue par au moins un hétérocycle condensé ou non avec un noyau phényle et comprenant au moins un atome d'azote quaternisé engagé dans ledit cycle et éventuellement au moins un autre hétéroatome (tel que l'oxygène, l'azote ou le soufre), éventuellement interrompue par au moins un groupement phényle ou naphtyle substitué ou non, éventuellement au moins un groupement ammonium quaternaire substitué par deux groupements alkyle en $C_1$-$C_{15}$ éventuellement substitués ; le bras de liaison ne comprenant pas de groupement nitro, nitroso ou peroxo.

Si les hétérocycles ou noyaux aromatiques sont substitués, ils le sont par exemple par un ou plusieurs radicaux alkyle en $C_1$-$C_8$ éventuellement substitués par un groupement hydroxy, alcoxy en $C_1$-$C_2$, hydroxyalcoxy en $C_2$-$C_4$, acétylamino, amino substitué par un ou deux radicaux alkyle en $C_1$-$C_4$, éventuellement porteurs d'au moins un groupement hydroxyle ou les deux radicaux pouvant former avec l'atome d'azote auquel ils sont rattachés, un hétérocycle à 5 ou 6 chaînons, comprenant éventuellement un autre hétéroatome identique ou différent de l'azote ; un atome d'halogène ; un groupement hydroxyle ; un radical alcoxy en $C_1$-$C_2$ ; un radical hydroxyalcoxy en $C_2$-$C_4$ ; un radical amino ; un radical amino substitué par un ou deux radicaux alkyle, identiques ou différents, en $C_1$-$C_4$ éventuellement porteurs d'au moins un groupement hydroxyle.

Parmi les colorants directs benzéniques utilisables selon l'invention, on peut citer de manière non limitative les composés suivants :

- 1,4-diamino-2-nitrobenzène,
- 1-amino-2 nitro-4-β-hydroxyéthylaminobenzène
- 1-amino-2 nitro-4-bis(β-hydroxyéthyl)-aminobenzène
- 1,4-bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-(β-hydroxyéthylamino-6-nitrobenzène
- 1-amino-2-nitro-4-β-hydroxyéthylamino-5-chlorobenzène
- 1,2-diamino-4-nitrobenzène
- 1-amino-2-β-hydroxyéthylamino-5-nitrobenzène
- 1,2-bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène

- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques, azométhines, méthines ou tétraazapentaméthines utilisables selon l'invention on peut citer les colorants cationiques décrits dans les demandes de brevets WO 95/15144, WO 95/01772 et EP 714954 ; FR 2189006, FR 2285851, FR-2140205, EP 1378544, EP 1674073.

Ainsi, on peut tout notamment citer les colorants suivants de formules (I) à (IV), et de préférence les composés de formules (I) et (III) :

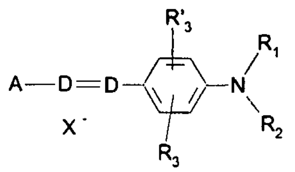

(I)

dans laquelle :

D représente un atome d'azote ou le groupement -CH,

$R_1$ et $R_2$, identiques ou différents, représentent un atome d'hydrogène ; un radical alkyle en $C_1$-$C_4$ pouvant être substitué par un radical -CN, -OH ou -$NH_2$ ou forment avec un atome de carbone du cycle benzénique un hétérocycle éventuellement oxygéné ou azoté, pouvant être substitué par un ou plusieurs radicaux alkyle en $C_1$-$C_4$ ; un radical 4'-aminophényle,

$R_3$ et $R'_3$, identiques ou différents, représentent un atome d'hydrogène ou d'halogène choisi parmi le chlore, le brome, l'iode et le fluor, un radical cyano, alkyl en $C_1$-$C_4$ , alcoxy en $C_1$-$C_4$ ou acétyloxy,

$X^-$ représente un anion de préférence choisi parmi le chlorure, le méthyl sulfate et l'acétate,

A représente un groupement choisi par les structures A1 à A18, de préférence A1, A4, A7, A13 et A18 suivantes :

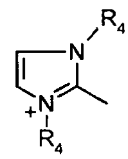

$A_1$

;

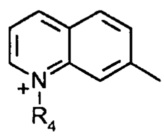

$A_2$

;

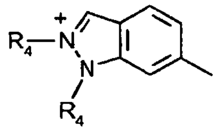

$A_3$

;

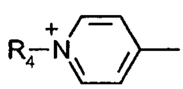

$A_4$

;

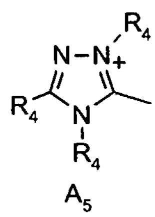

$A_5$

;

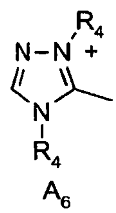

$A_6$

;

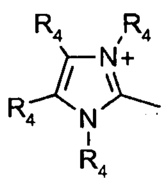

$A_7$

;

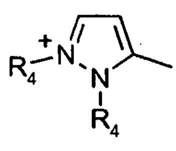

$A_8$

;

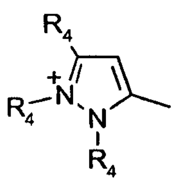

$A_9$

;

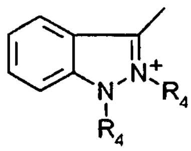 ; 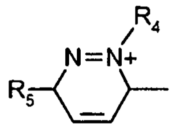 ; 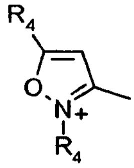 ;

$A_{10}$ $A_{11}$ $A_{12}$

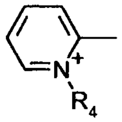 ; 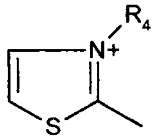 ; 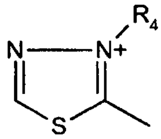 ;

$A_{13}$ $A_{14}$ $A_{15}$

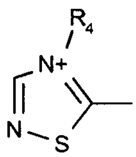 ; 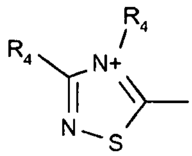 ; 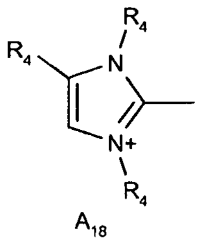

$A_{16}$ $A_{17}$ $A_{18}$

dans lesquelles $R_4$ représente un radical alkyle en $C_1$-$C_4$ pouvant être substitué par un radical hydroxyle et $R_5$ représente un radical alcoxy en $C_1$-$C_4$ ;

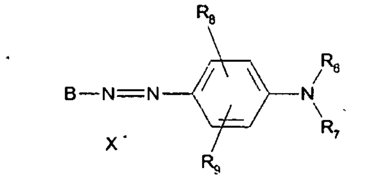 (II)

dans laquelle :

$R_6$ représente un atome d'hydrogène ou un radical alkyle en $C_1$-$C_4$,
$R_7$ représente un atome d'hydrogène, un radical alkyle pouvant être substitué par un radical -CN ou par un groupement amino, un radical 4'-aminophényle ou forme avec $R_6$ un hétérocycle éventuellement oxygéné et/ou azoté pouvant être substitué par un radical alkyle en $C_1$-$C_4$,
$R_8$ et $R_9$, identiques ou différents, représentent un atome d'hydrogène, un atome d'halogène tel que le brome, le chlore, l'iode ou le fluor, un radical alkyle en $C_1$-$C_4$ ou alcoxy en $C_1$-$C_4$, un radical - CN,
$X^-$ représente un anion de préférence choisi parmi le chlorure, le méthyl sulfate et l'acétate,
B représente un groupement choisi par les structures B1 à B6 suivantes :

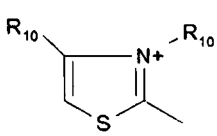 ; 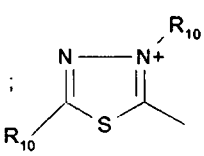 ; 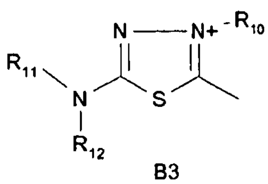 ;

B1 B2 B3

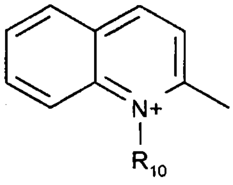 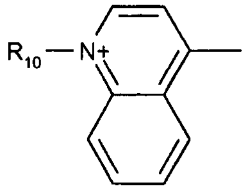 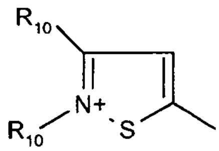

B4 B5 B6

dans lesquelles $R_{10}$ représente un radical alkyle en $C_1$-$C_4$, $R_{11}$ et $R_{12}$, identiques ou différents, représentent un atome d'hydrogène ou un radical alkyle en $C_1$-$C_4$ ;

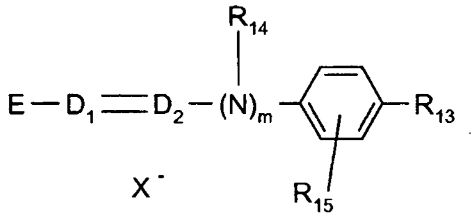

(III)

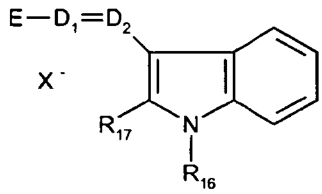

(III')

dans lesquelles :

$R_{13}$ représente un atome d'hydrogène, un radical alcoxy en $C_1$-$C_4$, un atome d'halogène tel que le brome, le chlore, l'iode ou le fluor,
$R_{14}$ représente un atome d'hydrogène, un radical alkyle en $C_1$-$C_4$ ou forme avec un atome de carbone du cycle benzénique un hétérocycle éventuellement oxygéné et/ou substitué par un ou plusieurs groupements alkyle en $C_1$-$C_4$,
$R_{15}$ représente un atome d'hydrogène ou d'halogène tel que le brome, le chlore, l'iode ou le fluor,
$R_{16}$ et $R_{17}$, identiques ou différents, représentent un atome d'hydrogène ou un radical alkyle en $C_1$-$C_4$,
$D_1$ et $D_2$, identiques ou différents, représentent un atome d'azote ou le groupement -CH,
m = 0 ou 1, de préférence 1,
étant entendu que lorsque $R_{13}$ représente un groupement amino non substitué, alors $D_1$ et $D_2$ représentent simultanément un groupement -CH et m = 0,
$X^-$ représente un anion de préférence choisi parmi le chlorure, le méthyl sulfate et l'acétate,
E représente un groupement choisi par les structures E1 à E8, plus particulièrement E1, E2 et E7, suivantes :

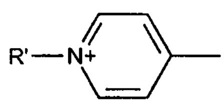 ; 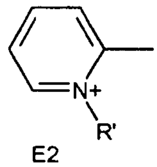 ;

E1 E2

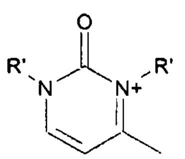 ; 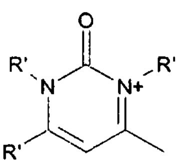 ; 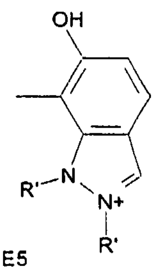 ;

E3 E4 E5

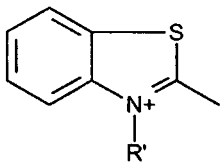 ; 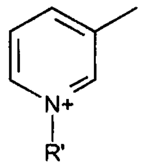 et 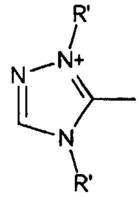

E6 E7 E8 ;

dans lesquelles R' représente un radical alkyle en $C_1$-$C_4$ ;
lorsque m = 0 et que $D_1$ représente un atome d'azote, alors E peut également désigner un groupement de structure E9 suivante :

E9 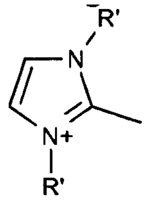 ;

dans laquelle R' représente un radical alkyle en $C_1$-$C_4$.

G-N=N-J (IV)

dans laquelle :

le symbole G représente un groupement choisi parmi les structures $G_1$ à $G_3$ suivantes :

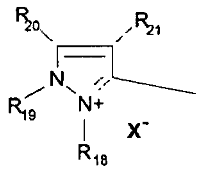 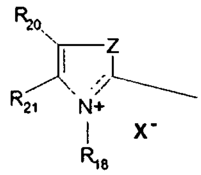 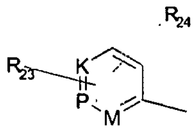

$G_1$ $G_2$ $G_3$

structures $G_1$ à $G_3$ dans lesquelles,

$R_{18}$ désigne un radical alkyle en $C_1$-$C_4$, un radical phényle pouvant être substitué par un radical alkyle en $C_1$-$C_4$ ou un atome d'halogène choisi parmi le chlore, le brome, l'iode et le fluor ;
$R_{19}$ désigne un radical alkyle en $C_1$-$C_4$ ou un radical phényle;
$R_{20}$ et $R_{21}$, identiques ou différents, représentent un radical alkyle en $C_1$-$C_4$ , un radical phényle, ou forment ensemble dans $G_1$ un cycle benzénique substitué par un ou plusieurs radicaux alkyle en $C_1$-$C_4$, alcoxy en $C_1$-$C_4$, ou $NO_2$, ou forment ensemble dans $G_2$ un cycle benzénique éventuellement substitué par un ou plusieurs radicaux alkyle en $C_1$-$C_4$ , alcoxy en $C_1$-$C_4$, ou $NO_2$;
$R_{20}$ peut désigner en outre un atome d'hydrogène;
Z désigne un atome d'oxygène, de soufre ou un groupement - $NR_{19}$;
M représente un groupement -CH, -CR (R désignant alkyle en $C_1$-$C_4$), ou $-NR_{22}(X^-)_r$;
K représente un groupement -CH, -CR (R désignant alkyle en $C_1$-$C_4$), ou $-NR_{22}(X^-)_r$;
P représente un groupement -CH, -CR (R désignant alkyle en $C_1$-$C_4$), ou $-NR_{22}(X^-)_r$; r désigne zéro ou 1;

$R_{22}$ représente un atome $O^-$, un radical alcoxy en $C_1$-$C_4$, ou un radical alkyle en $C_1$-$C_4$;
$R_{23}$ et $R_{24}$, identiques ou différents, représentent un atome d'hydrogène ou d'halogène choisi parmi le chlore, le brome, l'iode et le fluor, un radical alkyle en $C_1$-$C_4$, alcoxy en $C_1$-$C_4$, un radical - $NO_2$;
$X^-$ représente un anion de préférence choisi parmi le chlorure, l'iodure, le méthyl sulfate, l'éthyl sulfate, l'acétate et le perchlorate;
sous réserve que,
si $R_{22}$ désigne $O^-$, alors r désigne zéro;
si K ou P ou M désignent -N-alkyle $C_1$-$C_4$ $X^-$, alors $R_{23}$ ou $R_{24}$ est de préférence (avec le « ou non » cetet condition n'était plus utile ; en la conservant, on peut limiter le recouvrement enter els formules. Ceci dit, ce n'est aps d'une très grande importance je pense) différent d'un atome d'hydrogène;
si K désigne $-NR_{22}(X^-)_r$, alors M= P= -CH, -CR;
si M désigne $-NR_{22}(X^-)_r$, alors K= P= -CH, -CR;
si P désigne $-NR_{22}(X^\sim)_r$, alors K= M et désignent -CH ou -CR;
si Z désigne un atome de soufre avec $R_{21}$ désignant alkyle en $C_1$-$C_4$ , alors $R_{20}$ est différent d'un atome d'hydrogène;
si Z désigne $-NR_{22}$ avec $R_{19}$ désignant alkyle en $C_1$-$C_4$, alors au moins l'un des radicaux $R_{18}$, $R_{20}$ ou $R_{21}$ du groupement de structure $G_2$ est différent d'un radical alkyle en $C_1$-$C_4$;
le symbole J représente :

- (a) un groupement de structure $J_1$ suivante :

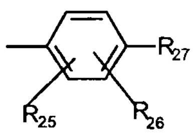

$J_1$

structure $J_1$ dans laquelle,

$R_{25}$ représente un atome d'hydrogène, un atome d'halogène choisi parmi le chlore, le brome, l'iode et le fluor, un radical alkyle en $C_1$-$C_4$, alcoxy en $C_1$-$C_4$, un radical -OH, $-NO_2$, $-NHR_{28}$, $-NR_{29}R_{30}$, - NHCOalkyle en $C_1$-$C_4$, ou forme avec $R_{26}$ un cycle à 5 ou 6 chaînons contenant ou non un ou plusieurs hétéroatomes choisis parmi l'azote, l'oxygène ou le soufre;
$R_{26}$ représente un atome d'hydrogène, un atome d'halogène choisi parmi le chlore, le brome, l'iode et le fluor, un radical alkyle en $C_1$-$C_4$, alcoxy en $C_1$-$C_4$,
ou forme avec $R_{27}$ ou $R_{28}$ un cycle à 5 ou 6 chaînons contenant ou non un ou plusieurs hétéroatomes choisis parmi l'azote, l'oxygène ou le soufre;
$R_{27}$ représente un atome d'hydrogène, un radical -OH, un radical $-NHR_{28}$, un radical $-NR_{29}R_{30}$;
$R_{28}$ représente un atome d'hydrogène, un radical alkyle en $C_1$-$C_4$, un radical monohydroxyalkyle en $C_1$-$C_4$, polyhydroxyalkyle en $C_2$-$C_4$, un radical phényle;
$R_{29}$ et $R_{30}$, identiques ou différents, représentent un radical alkyle en $C_1$-$C_4$, un radical monohydroxyalkyle en $C_1$-$C_4$, polyhydroxyalkyle en $C_2$-$C_4$;

- (b) un groupement hétérocyclique azoté à 5 ou 6 chaînons susceptible de renfermer d'autres hétéroatomes et/ou des groupements carbonylés et pouvant être substitué par un ou plusieurs radicaux alkyle en $C_1$-$C_4$, amino ou phényle,
et notamment un groupement de structure $J_2$ suivante :

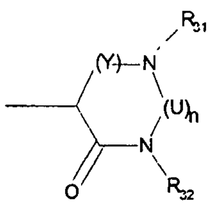

$J_2$

structure $J_2$ dans laquelle,

$R_{31}$ et $R_{32}$, identiques ou différents, représentent un atome d'hydrogène, un radical alkyle en $C_1$-$C_4$, un radical phényle;

Y désigne le radical -CO- ou le radical

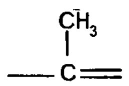

;

n = 0 ou 1, avec, lorsque n désigne 1, U désigne le radical - CO- .

Dans les structures (I) à (IV) définies ci-dessus le groupement alkyle ou alcoxy en $C_1$-$C_4$ désigne de préférence méthyle, éthyle, butyle, méthoxy, éthoxy.

Parmi les composés de formules (I) et (III), on préfère les composés suivants :

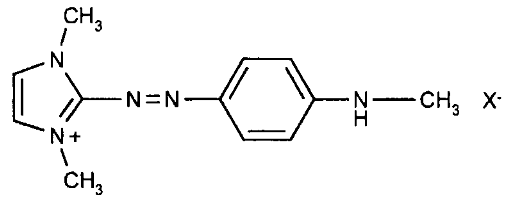

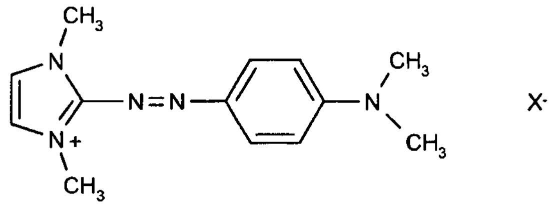

,

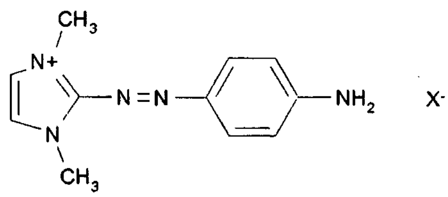

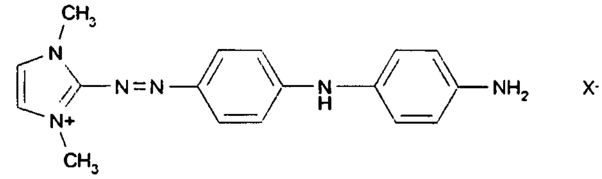

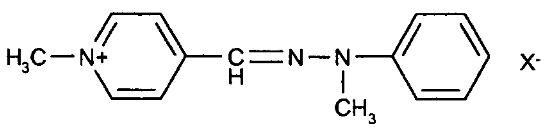

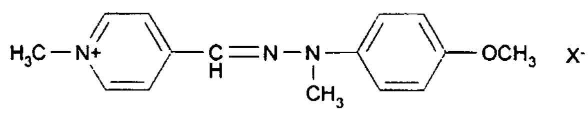

;

On peut également citer parmi les colorants directs azoïques les colorants suivants, décrits dans le COLOUR

INDEX INTERNATIONAL 3e édition :

- Disperse Red 17
- Basic Red 22
- Basic Red 76
- Basic Yellow 57
- Basic Brown 16
- Basic Brown 17
- Disperse Black 9.

On peut aussi citer le 1-(4'-aminodiphénylazo)-2-méthyl-4bis-(β-hydroxyéthyl)aminobenzène.

Parmi les colorants directs quinoniques on peut citer les colorants suivants :

- Disperse Red 15
- Solvent Violet 13
- Disperse Violet 1
- Disperse Violet 4
- Disperse Blue 1
- Disperse Violet 8
- Disperse Blue 3
- Disperse Red 11
- Disperse Blue 7
- Basic Blue 22
- Disperse Violet 15
- Basic Blue 99

ainsi que les composés suivants :

- 1-N-méthylmorpholiniumpropylamino-4-hydroxy anthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques, on peut citer les composés suivants :

- Basic Blue 17
- Basic Red 2.

Parmi les colorants triarylméthaniques utilisables selon l'invention, on peut citer les composés suivants :

- Basic Green 1
- Basic Violet 3
- Basic Violet 14
- Basic Blue 7
- Basic Blue 26

Parmi les colorants indoaminiques utilisables selon l'invention, on peut citer les composés suivants :

- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

Parmi les colorants de type tétraazapentaméthiniques utilisables selon l'invention, on peut citer les composés suivants figurant dans le tableau ci-dessous :

| | |
|---|---|
| 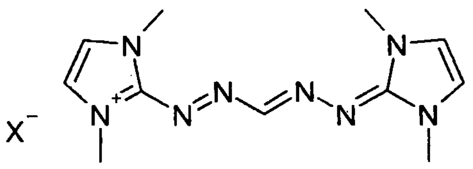 | 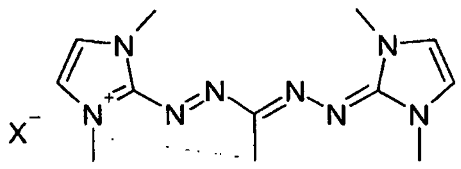 |
| 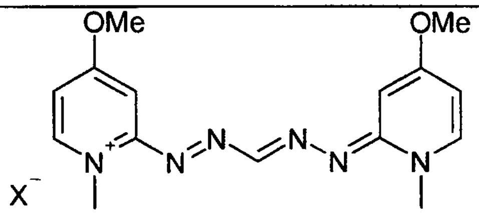 | 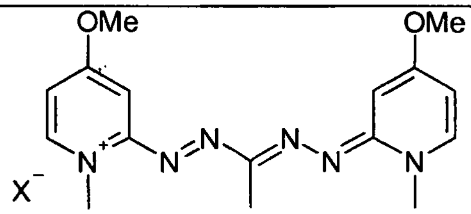 |
| 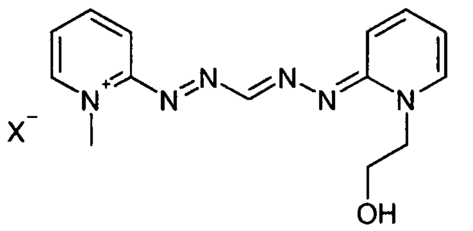 | 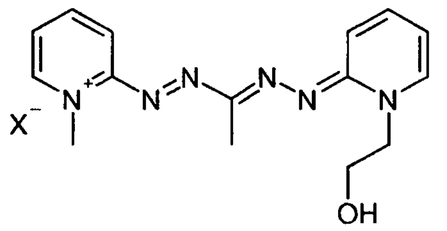 |

| | |
|---|---|
| 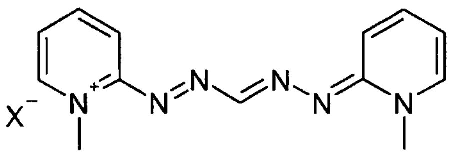 | 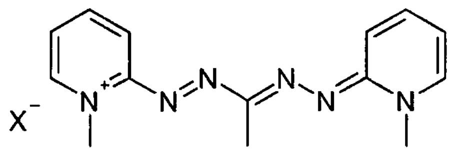 |
| 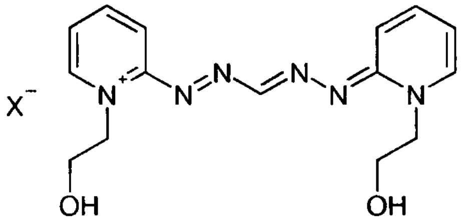 | 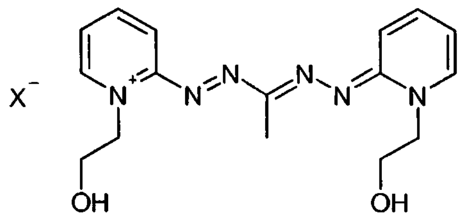 |

X⁻ représente un anion de préférence choisi parmi le chlorure, l'iodure, le méthyl sulfate, l'éthyl sulfate, l'acétate et le perchlorate.

Parmi les colorants polychromophoriques, on peut citer plus particulièrement les colorants di- ou tri- chromophoriques azoïques et/ou azométhiniques (hydrazoniques), symétriques ou non, comprenant d'une part au moins un hétérocycle aromatique comprenant 5 ou 6 chaînons, éventuellement condensé, comprenant au moins un atome d'azote quaternisé engagé dans ledit hétérocycle et éventuellement au moins un autre hétéroatome (tel que l'azote, le soufre, l'oxygène), et d'autre part, au moins un groupement phényle ou naphtyle, éventuellement substitué, éventuellement porteur d'au moins un groupement OR avec R représentant un atome d'hydrogène, un radical alkyle éventuellement substitué en $C_1$-$C_6$, un noyau phényle éventuellement substitué, ou d'au moins un groupement $N(R')_2$ avec R' identiques ou non, représentant un atome d'hydrogène, un radical alkyle éventuellement substitué en $C_1$-$C_6$, un noyau phényle éventuellement substitué ; les radicaux R' pouvant former avec l'atome d'azote auquel ils sont liés, un hétérocycle saturé

à 5 ou 6 chaînons, ou bien encore l'un et/ou les deux radicaux R' peuvent former chacun avec l'atome de carbone du cycle aromatique placé en ortho de l'atome d'azote, un hétérocycle saturé à 5 ou 6 chaînons.

A titre d'hétérocycle cationique aromatique, on peut citer de préférence, les cycles à 5 ou 6 chaînons comprenant 1 à 3 atomes d'azote, de préférence 1 ou 2 atomes d'azote, l'un étant quaternisé ; ledit hétérocycle étant par ailleurs éventuellement condensé à un noyau benzénique. Il est à noter de même que l'hétérocycle peut éventuellement comprendre un autre hétéroatome différent de l'azote, comme le soufre ou l'oxygène.

Si les hétérocycles ou groupements phényle ou napthyle sont substitués, ils le sont par exemple par un ou plusieurs radicaux alkyle en $C_1$-$C_8$ éventuellement substitués par un groupement hydroxy, alcoxy en $C_1$-$C_2$, hydroxyalcoxy en $C_2$-$C_4$, acétylamino, amino substitué par un ou deux radicaux alkyle en $C_1$-$C_4$, éventuellement porteurs d'au moins un groupement hydroxyle ou les deux radicaux pouvant former avec l'atome d'azote auquel ils sont rattachés, un hétérocycle à 5 ou 6 chaînons, comprenant éventuellement un autre hétéroatome identique ou différent de l'azote ; un atome d'halogène ; un groupement hydroxyle ; un radical alcoxy en $C_1$-$C_2$ ; un radical hydroxyalcoxy en $C_2$-$C_4$ ; un radical amino ; un radical amino substitué par un ou deux radicaux alkyle, identiques ou différents, en $C_1$-$C_4$ éventuellement porteurs d'au moins un groupement hydroxyle.

Ces polychromophores sont reliés entre eux au moyen d'au moins un bras de liaison comprenant éventuellement au moins un atome d'azote quaternisé engagé ou non dans un hétérocycle saturé ou non, éventuellement aromatique.

De préférence, le bras de liaison est une chaîne alkyle en $C_1$-$C_{20}$, linéaire, ramifiée ou cyclique, éventuellement interrompue par au moins un hétéroatome (tel que l'azote, l'oxygène) et/ou par au moins un groupe en comprenant (CO, $SO_2$), éventuellement interrompue par au moins un hétérocycle condensé ou non avec un noyau phényle et comprenant au moins un atome d'azote quaternisé engagé dans ledit cycle et éventuellement au moins un autre hétéroatome (tel que l'oxygène, l'azote ou le soufre), éventuellement interrompue par au moins un groupement phényle ou naphtyle substitué ou non, éventuellement au moins un groupement ammonium quaternaire substitué par deux groupements alkyle en $C_1$-$C_{15}$ éventuellement substitués ; le bras de liaison ne comprenant pas de groupement nitro, nitroso ou peroxo.

La liaison entre le bras de liaison et chaque chromophore se fait en général au moyen d'un hétéroatome substituant le noyau phényle ou napthyle ou au moyen de l'atome d'azote quaternisé de l'hétérocycle cationique.

Le colorant peut comprendre des chromophores identiques ou non.

A titre d'exemples de tels colorants, on pourra notamment se reporter aux demandes de brevets EP 1637566, EP 1619221, EP 1634926, EP 1619220, EP 1672033, EP 1671954, EP 1671955, EP 1679312, EP 1671951, EP167952, EP167971, WO 06/063866, WO 06/063867, WO 06/063868, WO 06/063869, EP 1408919, EP 1377264, EP 1377262, EP 1377261, EP 1377263, EP 1399425, EP 1399117, EP 1416909, EP 1399116, EP 1671560.

On peut aussi également mettre en oeuvre des colorants directs cationiques cités dans les demandes EP 1006153, qui décrit des colorants comprenant deux chromophores de type anthraquinones reliés au moyen d'un bras de liaison cationique ; EP 1433472, EP 1433474, EP 1433471 et EP 1433473 qui décrivent des colorants dichromophoriques identiques ou non, reliés par un bras de liaison cationique ou non, ainsi que EP 6291333 qui décrit notamment des colorants comprenant trois chromophores, l'un d'eux étant un chromophore anthraquinone auquel sont reliés deux chromophores de type azoïque ou diazacarbocyanine ou l'un de ses isomères.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine, les orcéines. On peut également utiliser les extraits ou décoctions contenant ces colorants naturels et notamment les cataplasmes ou extraits à base de henné.

Lorsqu'ils sont présents, le ou les colorants directs représentent plus particulièrement de 0,0001 à 10 % en poids du poids total de la composition, et de préférence de 0,005 à 5 % en poids.

La composition cosmétique (B) peut comprendre l'un et/ou l'autre types de colorants. Elle peut éventuellement être issue du mélange de deux compositions colorantes l'une comprenant le ou les colorants d'oxydation, l'autre, le ou les colorants directs.

La composition cosmétique (B) comprend en outre un ou plusieurs agents alcalins, de préférence choisi parmi les amines organiques et leurs sels, les bases organiques et les sels d'ammonium.

L'agent alcalin peut être organique ou minéral ou hybride.

Un premier type d'agents alcalins utilisables au sens de la présente invention sont les amines organiques dont le pKb à 25°C est inférieur à 12, et de préférence inférieur à 10, encore plus avantageusement inférieur à 6. Il est à noter qu'il s'agit du pKb correspondant à la fonction de basicité la plus élevée.

Selon une première variante de l'invention, l'amine organique comprend une fonction amine primaire, secondaire ou tertiaire, et un ou plusieurs groupements alkyle, linéaires ou ramifiés, en $C_1$-$C_8$ porteurs d'un ou plusieurs radicaux hydroxyle.

Conviennent en particulier à la réalisation de l'invention les amines organiques choisies parmi les alcanolamines telles que les mono, di- ou tri- alcanolamines, comprenant un à trois radicaux hydroxyalkyle, identiques ou non, en $C_1$-$C_4$.

Parmi des composés de ce type, on peut citer la monoéthanolamine, la diéthanolamine, la triéthanolamine, la

monoisopropanolamine, la diisopropanolamine, la N-diméthylaminoéthanolamine, le 2-amino-2-méthyl-1-propanol, la triisopropanolamine, le 2-amino-2-méthyl-1,3-propanediol, le 3-amino-1,2-propanediol, le 3-diméthylamino-1,2-propanediol, le tris-hydroxyméthylaminométhane.

Conviennent également les amines organiques de formule suivante :

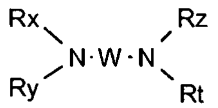

dans laquelle W est un reste alkylène en $C_1$-$C_6$ éventuellement substitué par un groupement hydroxyle ou un radical alkyle en $C_1$-$C_6$ ; Rx, Ry, Rz et Rt, identiques ou différents, représentent un atome d'hydrogène, un radical alkyle en $C_1$-$C_6$ ou hydroxyalkyle en $C_1$-$C_6$, aminoalkyle en $C_1$-$C_6$.

On peut citer à titre d'exemple de telles amines, le 1,3 diaminopropane, le 1,3 diamino-2-propanol, la spermine, la spermidine.

Selon une autre variante de l'invention, l'amine organique est choisie parmi les acides aminés.

Plus particulièrement, les acides aminés utilisables sont d'origine naturelle ou de synthèse, sous leur forme L, D, ou racémique et comportent au moins une fonction acide choisie plus particulièrement parmi les fonctions acides carboxyliques, sulfoniques, phosphoniques ou phosphoriques. Les acides aminés peuvent se trouver sous forme neutre ou ionique.

De manière avantageuse, les acides aminés sont des acides aminés basiques comprenant une fonction amine supplémentaire éventuellement incluse dans un cycle ou dans une fonction uréido.

De tels acides aminés basiques sont choisis de préférence parmi ceux répondant à la formule (I) suivante :

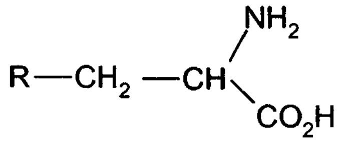

**(I)**

où R désigne un groupe choisi parmi :

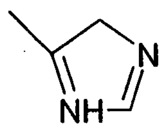

- $(CH_2)_3NH_2$ ;
- $(CH_2)_2NH_2$ ; $-(CH_2)_2NHCONH_2$ ;

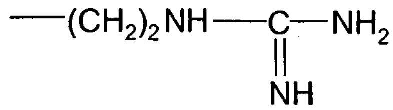

Les composés correspondants à la formule (I) sont l'histidine, la lysine, l'arginine, l'ornithine, la citrulline.

A titre d'acides aminés utilisables dans la présente invention, on peut notamment citer l'acide aspartique, l'acide glutamique, l'alanine, l'arginine, l'ornithine, la citrulline, l'asparagine, la carnitine, la cystéine, la glutamine, la glycine, l'histidine, la lysine, l'isoleucine, la leucine, la méthionine, la N-phénylalanine, la proline, la serine, la taurine, la thréonine, le tryptophane, la tyrosine et la valine.

Selon une variante préférée de l'invention, l'amine organique est choisie parmi les acides aminés basiques. Les acides aminés particulièrement préférés sont l'arginine, la lysine, l'histidine, ou leurs mélanges.

Selon une autre variante de l'invention, l'amine organique est choisie parmi les amines organiques de type hétérocycliques. On peut en particulier citer, outre l'histidine déjà mentionnée dans les acides aminés, la pyridine, la pipéridine, l'imidazole, le triazole, le tétrazole, le benzimidazole .

Selon une autre variante de l'invention, l'amine organique est choisie parmi les dipeptides d'acides aminés. A titre de dipeptides d'acides aminés utilisables dans la présente invention, on peut notamment citer la carnosine, l'anserine et la baleine

Selon une autre variante de l'invention, l'amine organique est choisie parmi les composés comportant une fonction guanidine. A titre d'amines de ce type utilisables dans la présente invention, on peut notamment citer outre l'arginine déjà mentionnée à titre d'acide aminé, la créatine, la créatinine, la 1,1-diméthylguanidine, 1,1-diéthylguanidine, la glycocyamine, la metformin, l'agmatine, la n-amidinoalanine, l'acide 3-guanidinopropionique, l'acide 4-guanidinobutyrique et l'acide 2-([amino(imino)methyl]amino)ethane-1-sulfonique.

De préférence l'amine organique est une alcanolamine. Plus préférentiellement l'amine organique est choisie parmi le 2-amino 2-méthyl 1-propanol, la monoéthanolamine ou leurs mélanges. Encore plus préférentiellement l'amine organique est la monoéthanolamine.

Un deuxième type d'agents alcalins utilisables au sens de la présente invention comprend les sels organiques ou inorganiques (dans ce cas on parle d'agents alcalins hybrides) des amines organiques telles que décrites ci-dessus

De préférence, les sels organiques sont choisis parmi les sels d'acides organiques tels que les citrates, les lactates, les glycolates, les gluconates, les acétates, les propionates, les fumarates, les oxalates et les tartrates.

De préférence, les sels inorganiques sont choisis parmi les halogénohydrates (chlorhydrates par exemple), les carbonates, les hydrogénocarbonates, les sulfates, les hydrogénophosphates et les phosphates.

Un troisième type d'agents alcalins utilisables au sens de la présente invention sont les bases inorganiques.

Par composé inorganique, au sens de la présente invention, on entend tout composé possédant dans sa structure un ou plusieurs éléments des colonnes 1 à 13 du tableau périodique des éléments autres que l'hydrogène.

Selon un mode de réalisation particulier de l'invention, la base inorganique contient un ou plusieurs éléments des colonnes 1 et 2 du tableau périodique des éléments autre que l'hydrogène.

Dans une variante préférée la base inorganique présente la structure suivante :

$$(Z_1^{x-})_m(Z_2^{y+})_n$$

Dans laquelle :

$Z_2$ désigne un métal des colonnes 1 à 13 du tableau périodique des éléments, de préférence 1 ou 2, comme le sodium ou le potassium ;
$Z_1^{x-}$ désigne un anion choisi parmi les ions $CO_3^{2-}$, $OH^-$, $HCO_3^{2-}$, $SiO_3^{2-}$, $HPO_4^{2-}$, $PO_4^{3-}$, $B_4O_7^{2-}$, de préférence parmi les ions $CO_3^{2-}$, $OH^-$, $SiO_3^{2-}$ ;
x désigne 1 , 2 ou 3 ;
y désigne 1, 2, 3 ou 4 ;
m et n désignent indépendamment l'un de l'autre 1, 2, 3 ou 4 ;
avec n.y=m.x.

De préférence, la base inorganique correspond à la formule suivante $(Z_1^{x-})_m(Z_2^{y+})_n$, dans laquelle $Z_2$ désigne un métal des colonnes 1 et 2, du tableau périodique des éléments ; $Z_1^{x-}$ désigne un anion choisi parmi les ions $CO_3^{2-}$, $OH^-$, $SiO_3^{2-}$, x vaut 1, y désigne 1 ou 2, m et n désignent indépendamment l'un de l'autre 1 ou 2 avec n.y=m.x.

A titre de base inorganique utilisable selon l'invention on peut citer, le carbonate de sodium, le carbonate de potassium, la soude, la potasse, le métasilicate de sodium, le métasilicate de potassium.

Un quatrième type d'agents alcalins utilisables au sens de la présente invention sont les sels d'ammonium.

Les sels d'ammonium utilisables dans la composition B selon la présente invention sont les sels d'ammonium ($NH_4^+$).

Les sels d'ammonium utilisables dans la composition B selon la présente invention sont de préférence choisi parmi les sels d'acide suivants : acétate, carbonate, bicarbonate, chlorure, citrate, nitrate, nitrite, phosphate, sulfate. De manière particulièrement préférée, le sel est le carbonate. De manière particulièrement préférée on utilisera le carbonate d'ammonium.

De manière avantageuse, la composition (B) présente une teneur en agents alcalins allant de 0,01 à 30 % en poids, de préférence de 0,1 à 20 % en poids par rapport au poids de ladite composition.

Il est à noter que, de préférence, la composition (B) ne comprend pas d'ammoniaque en tant qu'agent alcalin. Si toutefois elle en comprenait, sa teneur ne dépasserait pas de préférence 0,03 % en poids (exprimé en $NH_3$), et mieux ne dépasserait pas 0,01 % en poids par rapport au poids de ladite composition finale.

La composition (B) peut être une composition anhydre ou aqueuse. Par composition aqueuse, on entend une composition comprenant plus de 5 % en poids d'eau, de préférence plus de 10 % en poids d'eau, et de manière encore plus avantageuse plus de 20 % en poids d'eau.

De préférence, la composition cosmétique (B) est une composition aqueuse.

De préférence la composition (B) contient de l'eau. Encore plus préférentiellement la concentration en eau peut aller de 10 à 90 %, mieux de 20 à 80 % du poids total de la composition.

La composition cosmétique (B) peut éventuellement comprendre un ou plusieurs solvants.

A titre de solvant organique, on peut par exemple citer, les alcanols, linéaires ou ramifiés, en $C_2$-$C_4$, tels que l'éthanol et l'isopropanol ; le glycérol ; les polyols et éthers de polyols comme le 2-butoxyéthanol, le propylèneglycol, le dipropylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Le ou les solvants, s'ils sont présents, représentent une teneur allant habituellement de 1 à 40 % en poids par rapport au poids de la composition cosmétique (B), et de préférence allant de 5 à 30 % en poids.

La composition cosmétique (B) peut également comprendre des additifs classiques tels que ceux qui ont été listés auparavant et l'on pourra s'y reporter.

Le pH de la composition cosmétique (B) si elle est aqueuse est compris entre 2 et 12, de préférence entre 8 et 11. Le pH est adapté en utilisant des agents acidifiants ou alcalinisants

Parmi les agents acidifiants, on peut citer à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

En ce qui concerne l'agent alcalinisant, s'il est présent, il peut être choisi parmi les amines organiques non salifiées décrites auparavant, ou éventuellement, bien que non préférentiellement, l'ammoniaque. De préférence, si la composition comprend de l'ammoniaque ou un de ses sels, alors la quantité d'agent(s) alcalinisant(s) est supérieure à celle de l'ammoniaque, exprimée en $NH_3$. Si l'ammoniaque était employée en tant qu'agent alcalinisant dans la composition (B), alors la teneur en ammoniaque de la composition (B) ne dépasserait pas de préférence 0,03 % en poids (exprimé en $NH_3$), et mieux ne dépasserait pas 0,01 % en poids par rapport au poids de ladite composition finale.

Avantageusement, la teneur en ammoniaque de la composition finale, appliquée sur les cheveux, ne dépasse pas 0,03 % en poids (exprimé en $NH_3$), et mieux ne dépasse pas 0,01 % en poids par rapport au poids de ladite composition finale.

Il est indiqué que la composition finale résulte du mélange des compositions (A), (B) et (C) ; ces mélanges étant réalisés avant application sur les fibres kératiniques (préparation extemporanée) le procédé est mis en oeuvre au moyen d'une préparation extemporanée obtenue en mélangeant les compositions (A), (B) et (C); la durée entre le mélange et l'application sur les cheveux n'excède pas de préférence 30 minutes, de préférence 10 minutes, de manière encore préférée 5 minutes

Enfin, le procédé est mis en oeuvre avec une composition (C) comprenant un ou plusieurs agents oxydants.

Plus particulièrement, le ou les agents oxydants sont choisis parmi le peroxyde d'hydrogène, le peroxyde d'urée, les bromates ou ferricyanures de métaux alcalins, les sels peroxygénés comme par exemple les persulfates, les perborates et les percarbonates de métaux alcalins ou alcalino-terreux, ainsi que les peracides et leurs précurseurs. On peut également utiliser à titre d'agent oxydant une ou plusieurs enzymes d'oxydo-réduction telles que les laccases, les peroxydases et les oxydoréductases à 2 électrons (telles que l'uricase), éventuellement en présence de leur donneur ou cofacteur respectif.

De préférence la composition finale ne contient pas de peracides et leurs précurseurs, de sels peroxygénés comme par exemple les persulfates, les perborates, les percarbonates de métaux alcalins ou alcalino-terreux.

De préférence, le ou les agents oxydants sont choisis parmi le peroxyde d'hydrogène, le peroxyde d'urée, les bromates ou ferricyanures de métaux alcalins.

Cet agent oxydant est avantageusement constitué par du peroxyde d'hydrogène et notamment en solution aqueuse (eau oxygénée) dont la concentration peut varier, plus particulièrement de 0,1 à 50% en poids, et encore plus préférentiellement de 0,5 à 20% en poids, mieux de 1 à 15% en poids par rapport à la composition oxydante (C).

En fonction du degré d'éclaircissement souhaité, l'agent oxydant peut également comprendre un agent oxydant choisi de préférence parmi les sels peroxygénés.

La composition oxydante (C) peut être aqueuse ou non. Par composition aqueuse, on entend une composition comprenant plus de 5 % en poids d'eau, de préférence plus de 10 % en poids d'eau, et de manière encore plus avantageuse plus de 20 % en poids d'eau.

De préférence, la composition oxydante (C) est une composition aqueuse.

Elle peut également comprendre un ou plusieurs solvants organiques.

A titre de solvant organique, on peut par exemple citer, les alcanols, linéaires ou ramifiés, en $C_2$-$C_4$, tels que l'éthanol et l'isopropanol ; le glycérol ; les polyols et éthers de polyols comme le 2-butoxyéthanol, le propylèneglycol, le dipropylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Le ou les solvants, s'ils sont présents, représentent une teneur allant habituellement de 1 à 40 % en poids par rapport au poids de la composition oxydante (C), et de préférence de 5 à 30 % en poids.

La composition oxydante (C) peut comprendre un ou plusieurs agents acidifiants décrits précédemment.

Habituellement, le pH de la composition oxydante (C), lorsqu'elle est aqueuse, est inférieur à 7.

La composition oxydante (C) peut également renfermer d'autres ingrédients classiquement employés dans le domaine, comme notamment ceux détaillés auparavant dans le cadre de la composition aqueuse (A) ou de la composition

(B).

Enfin, la composition oxydante (C) se présente sous diverses formes, comme par exemple une solution, une émulsion ou un gel.

On applique sur les fibres kératiniques, sèches ou humides, une composition obtenue par mélange extemporané avant l'application, des compositions (A), (B) et (C).

Avantageusement, la durée entre le mélange et l'application sur les cheveux n'excède pas de préférence 30 minutes, de préférence 10 minutes, de manière encore préférée 5 minutes

les rapports pondéraux R1 des quantités de compositions (A) + (B) / (C) et R2 des quantités de compositions (A) / (B) varient de 0,1 à 10, et de préférence de 0,3 à 3.

la composition obtenue après mélange des compositions (A), (B) et (C) décrites précédemment est telle que, après mélange, la quantité de corps gras est supérieure à 20% en poids, de préférence supérieure à 25% en poids et de manière encore plus avantageuse supérieure à 30% en poids.

En outre, le mélange présent sur les fibres (résultant du mélange extemporané des compositions) est laissé en place pour une durée, en général, de l'ordre de 1 minute à 1 heure, de préférence de 5 minutes à 30 minutes.

La température durant le procédé est classiquement comprise entre la température ambiante (entre 15 à 25°C) et 80°C, de préférence entre la température ambiante et 60°C.

A l'issue du traitement, les fibres kératiniques humaines sont éventuellement rincées à l'eau, subissent éventuellement un lavage avec un shampooing suivi d'un rinçage à l'eau, avant d'être séchées ou laissées à sécher.

Les exemples suivants servent à illustrer l'invention sans toutefois présenter un caractère limitatif.

## EXEMPLES

**Exemple de procédé d'éclaircissement** (hors invention)

On prépare les compositions suivantes dans lesquelles les quantités sont exprimées en grammes.

Compositions aqueuses (A) :

| | A1 | A2 |
|---|---|---|
| Mono-laurate de sorbitane oxyéthyléné (4OE) | 21,7 | 21,7 |
| Silice pyrogénée à caractère hydrophobe | 11,1 | 11,1 |
| Eau déminéralisée | 5 | 10 |
| Huile vaseline | qsp 100 | qsp 100 |

Composition (B)

| | |
|---|---|
| Monoéthanolamine pure | 40 |
| Eau déminéralisée | qsp 100 |

Au moment de l'emploi, on mélange :

- 9 parties en poids d'une composition aqueuse A (A1, ou A2)
- 1 partie en poids de la composition (B)
- 10 parties en poids d'oxydant Platinum international 20 volume (6% en peroxyde d'hydrogène) (C).

Le mélange résultant présente un pH de 10 + 0,1. Ce mélange est appliqué sur une mèche naturelle (hauteur de ton 4) selon un rapport de bain mélange/mèche 10/1 (g/g).

Le temps de pause est de 30 minutes à température ambiante (environ 27°C).

A l'issue de ce temps de pause, la mèche est rincée, puis lavée avec du shampooing Elsève multivitamines.

Résultats

La couleur des mèches a été évaluée dans le système CIE L* a* b*, au moyen d'un colorimètre Minolta Spectrophotometer CM2600D.

L'éclaircissement d'une mèche ($\Delta E_{ab}$*) a été évaluée dans le système CIE L* a* b*. Dans ce système L* a* b*, L* représente l'intensité de la couleur, a* indique l'axe de couleur vert/rouge et b* l'axe de couleur bleu/jaune. Plus la valeur de L* est faible, plus la couleur est foncée ou très intense.

Dans le tableau ci-dessous, on calcule la valeur de $\Delta E_{ab}$* à partir des valeurs de L*a*b* selon l'équation suivante (i) :

$$\Delta E_{ab}* = \sqrt{(L* - L_o*)^2 + (a* - a_o*)^2 + (b* - b_o*)^2} \quad (i)$$

L'éclaircissement d'une mèche ($\Delta E_{ab}$*) a été calculé sur des mèches de cheveux naturels de hauteur de ton 4.

Dans l'équation (i), L*, a* et b* représentent les valeurs mesurées sur des mèches de cheveux naturels de hauteur de ton 4 après traitement éclaircissant, et $L_0$*, $a_0$* et $b_0$* représentent les valeurs mesurées sur des mèches de cheveux naturels de hauteur de ton 4 sans traitement.

Plus la valeur de $\Delta E_{ab}$* est grande, plus important est l'éclaircissement (variation de couleur).

| | **L*** | **a*** | **b*** | **ΔE *ab** |
|---|---|---|---|---|
| Mèche naturelle (HT4) non traitée | **17.7** | **1.7** | **1.4** | **-** |
| Mèche traitée avec le procédé selon l'invention mettant en oeuvre la composition A1 | **23** | **6.6** | **7.7** | **9.6** |
| Mèche traitée avec le procédé selon l'invention mettant en oeuvre la composition A2 | **22.3** | **6.6** | **7.4** | **9** |

Le tableau ci-dessus montre que le procédé selon l'invention mis en oeuvre avec une composition comprenant un agent alcalin différent de l'ammoniaque permet d'obtenir un éclaircissement des fibres kératiniques.

**Exemple de procédé de coloration**

On prépare les compositions suivantes (quantités exprimées en grammes)

Composition aqueuse A :

| | 100g |
|---|---|
| Monolaurate de sorbitane oxyethyléné (40E) | 21,7 |
| Silice pyrogénée à caractère hydrophobe | 11,1 |
| Eau déminéralisée | 10 |
| Huile de vaseline | Qsp 100 |

Composition cosmétique B :

| | 100g |
|---|---|
| Para-phénylène diamine | 6,55 |
| Résorcine | 4,95 |
| 2-méthylrésorcine | 1,86 |
| 1-beta-hydroxyethyloxy-2,4-diamino-benzène dichlorhydrate | 0,15 |
| Métabisulfite de sodium en poudre | 0,46 |
| Acide erythorbique | 0,31 |
| monoéthanolamine | 40,07 |
| Eau déminéralisée | Qsp 100 |

Composition (C)

| | |
|---|---|
| Alcools gras | 2,28 |
| Tensioactifs non-ioniques | 1,42 |
| glycérine | 0,5 |
| peroxyde d'hydrogène | 6 |
| Stabilisants du peroxyde d'hydrogène | 0.12 |
| Eau déminéralisée | qsp 100 |

Au moment de l'emploi, on mélange :

- 9 parties en poids de la composition aqueuse (A)
- 1 parties en poids de la composition (B)
- 10 parties en poids de la composition aqueuse oxydante (C) de pH égal à 2,3.

Le mélange obtenu, dont le pH est environ 10 (± 0,1), est ensuite appliqué sur une mèche de cheveux naturels à 90% blancs (BN) et sur une mèche de cheveux à 90% blancs permanentés (BP). Le rapport de bain « mélange/mèche » est respectivement de 10/1 (g/g).

Le temps de pause est de 30 minutes à 27°C. A l'issue de ce temps, les mèches sont rincées, puis lavées avec du shampooing Elsève multivitamines.

Résultats

La couleur des mèches a été évaluée dans le système CIE L* a* b*, au moyen d'un colorimètre Minolta Spectrophotometer CM2600D.

Calcul de la sélectivité

On calcule la valeur de ΔE (sélectivité) à partir des valeurs de L* a* b* mesurées selon l'équation suivante (ii) :

$$\Delta E = \sqrt{(L^* - L_o^*)^2 + (a^* - a_o^*)^2 + (b^* - b_o^*)^2} \quad (ii)$$

Dans l'équation (ii), L*, a* et b* représentent les valeurs mesurées sur des mèches de cheveux blancs naturels colorés, et $L_0$*, $a_0$* et $b_0$* représentent les valeurs mesurées sur des mèches de cheveux blancs permanentés colorés.

La sélectivité de la coloration ΔE correspond à la variation de la couleur entre des cheveux naturels, représentatifs de la nature des cheveux à la racine, et des cheveux permanentés qui sont représentatifs de la nature des cheveux à la pointe. Plus la valeur de ΔE est faible, plus la coloration est homogène entre la pointe et la racine des cheveux.

Les résultats sont donnés dans le tableau ci-dessous.

| | L* | a* | b* | ΔE Sélectivité |
|---|---|---|---|---|
| Mèche de cheveu naturelle traitée avec la composition selon l'invention | 19,41 | 2,14 | 3,32 | 2,99 |
| Mèche de cheveu permanentée traitée avec la composition selon l'invention | 17,08 | 1,22 | 1,67 | |

Comme le montre le tableau ci-dessus, avec le procédé selon l'invention, on obtient une coloration puissante et peu sélective.

De plus, on n'observe aucune odeur agressive, ni pendant la préparation du mélange colorant, ni pendant le temps de pose sur mèches.

## Revendications

1. Procédé coloration des fibres kératiniques, en particulier des fibres kératiniques humaines telles que les cheveux, **caractérisé en ce qu'**il consiste à mélanger extemporanément avant application, des compositions (A), (B) et (C) :

   a) la composition cosmétique aqueuse (A) comprenant :

      - un ou plusieurs corps gras choisis parmi l'huile de vaseline, les polydécènes. les esters d'acides gras et/ou d'alcools gras, liquides ou leurs mélanges, et
      - un ou plusieurs tensioactifs ;

   la quantité des corps gras de la composition (A) étant au moins supérieure à 25% en poids par rapport au poids total de la composition (A) ;
   b) la composition cosmétique (B) comprenant - un ou plusieurs agents alcalins - et un ou plusieurs colorants d'oxydation et/ou un ou plusieurs colorants directs ;
   c) la composition (C) comprenant un ou plusieurs agents oxydants, puis

   à appliquer sur lesdites fibres kératiniques sèches ou humides. la composition ainsi obtenue,
   la composition obtenue après mélange étant telle que la quantité de corps gras est supérieure à 20% en poids.

2. Procédé selon la revendication précédente, **caractérisé en ce que** la composition cosmétique aqueuse (A) comprend plus de 5 % en poids d'eau, de préférence plus de 10%, encore plus particulièrement plus de 20% en poids d'eau.

3. Procédé selon l'une quelconque des revendications précédentes, **caractérisé en ce que** le ou les corps gras sont choisis parmi les composés liquides ou pâteux à température ambiante et à pression atmosphérique.

4. Procédé selon l'une quelconque des revendications précédentes, **caractérisé en ce que** la concentration en corps gras est de 25 à 80 % en poids, et de préférence de 25 à 65 % en poids, par rapport au poids total de la composition (A).

5. Procédé selon l'une quelconque des revendications précédentes, **caractérisé en ce que** la composition aqueuse (A) comprend un ou plusieurs tensioactifs choisis parmi les tensioactifs non ioniques en particulier les tensioactifs non ioniques mono- ou poly-oxyalkylénés et mono- ou poly-glycérolés.

6. Procédé selon l'une quelconque des revendications précédentes, **caractérisé en ce que** le ou les agents alcalins sont choisis parmi les amines organiques et leurs sels, les bases inorganiques et les sels d'ammonium.

7. Procédé selon la revendication précédente, **caractérisé en ce que** l'agent alcalin est la monoéthanolamine.

8. Procédé selon l'une quelconque des revendications précédentes, **caractérisé en ce que** la composition (B) présente une teneur en agents alcalins allant de 0,01 à 30 % en poids, de préférence de 0,1 à 20 % en poids par rapport au poids de ladite composition.

9. Procédé selon l'une quelconque des revendications précédentes, **caractérisé en ce que** la composition aqueuse (C) comprend un ou plusieurs agents oxydants choisis parmi le peroxyde d'hydrogène, le peroxyde d'urée, les bromates ou ferricyanures de métaux alcalins.

## Patentansprüche

1. Verfahren zum Färben von Keratinfasern, insbesondere menschlichen Keratinfasern, wie die Haare, **dadurch gekennzeichnet, dass** man bei dem Verfahren kurz vor der Anwendung die Zusammensetzungen (A), (B) und (C) mischt, wobei:

   a) die wässrige Kosmetikzusammensetzung (A) Folgendes umfasst:

      - ein oder mehrere Fette, ausgewählt aus Vaselineöl, Polydecenen, Estern von Fettsäuren und/oder Fettalkoholen, die flüssig sind, oder deren Gemische, und

- ein oder mehrere Tenside;

wobei die Menge an Fetten der Zusammensetzung (A) mindestens größer als 25 Gew.-%, bezogen auf das Gesamtgewicht der Zusammensetzung (A), ist;
(b) die Kosmetikzusammensetzung (B) Folgendes umfasst

- ein oder mehrere alkalische Mittel
- und einen oder mehrere Oxidationsfarbstoffe und/oder einen oder mehrere Direktfarbstoffe;

(c) die Zusammensetzung (C) ein oder mehrere Oxidationsmittel umfasst, und
die so erhaltene Zusammensetzung auf die trockenen oder feuchten Keratinfasern aufträgt,
wobei die nach dem Mischen erhaltene Zusammensetzung derart ist, dass die Menge an Fett größer als 20 Gew.-% ist.

**2.** Verfahren nach dem vorhergehenden Anspruch, **dadurch gekennzeichnet, dass** die wässrige Kosmetikzusammensetzung (A) mehr als 5 Gew.-% Wasser, vorzugsweise mehr als 10 Gew.-%, noch stärker bevorzugt mehr als 20 Gew.-% Wasser umfasst.

**3.** Verfahren nach einem der vorhergehenden Ansprüche, **dadurch gekennzeichnet, dass** das Fett oder die Fette aus Verbindungen ausgewählt ist/sind, die bei Umgebungstemperatur und Atmosphärendruck flüssig oder pastös sind.

**4.** Verfahren nach einem der vorhergehenden Ansprüche, **dadurch gekennzeichnet, dass** die Konzentration an Fett 25 bis 80 Gew.-% und vorzugsweise 25 bis 65 Gew.-%, bezogen auf das Gesamtgewicht der Zusammensetzung (A), beträgt.

**5.** Verfahren nach einem der vorhergehenden Ansprüche, **dadurch gekennzeichnet, dass** die wässrige Zusammensetzung (A) ein oder mehrere Tenside umfasst, ausgewählt aus nichtionischen Tensiden und insbesondere nichtionischen mono- oder polyoxyalkylenierten und mono- oder polyglycerinierten Tensiden.

**6.** Verfahren nach einem der vorhergehenden Ansprüche, **dadurch gekennzeichnet, dass** das oder die alkalische(n) Mittel aus organischen Aminen und deren Salzen, anorganischen Basen und Ammoniumsalzen ausgewählt ist/sind.

**7.** Verfahren nach dem vorhergehenden Anspruch, **dadurch gekennzeichnet, dass** das alkalische Mittel Monoethanolamin ist.

**8.** Verfahren nach einem der vorhergehenden Ansprüche, **dadurch gekennzeichnet, dass** die Zusammensetzung (B) einen Gehalt an alkalischen Mitteln von 0,01 bis 30 Gew.-%, vorzugsweise von 0,1 bis 20 Gew.-%, bezogen auf das Gewicht dieser Zusammensetzung, aufweist.

**9.** Verfahren nach einem der vorhergehenden Ansprüche, **dadurch gekennzeichnet, dass** die wässrige Zusammensetzung (C) ein oder mehrere Oxidationsmittel umfasst, ausgewählt aus Wasserstoffperoxid, Harnstoffperoxid, Alkalimetallbromaten oder - ferricyaniden.

## Claims

**1.** Process for dyeing keratin fibres, in particular human keratin fibres such as the hair, **characterized in that** it consists in mixing extemporaneously, before application, compositions (A), (B) and (C):

a) the aqueous cosmetic composition (A) comprising:

- one or more fatty substances chosen from liquid petroleum jelly, polydecenes and liquid esters of fatty acids and/or of fatty alcohols, or mixtures thereof, and
- one or more surfactants;
- the amount of fatty substances of the composition (A) being at least greater than 25% by weight compared to the total weight of the composition (A) ;

b) the cosmetic composition (B) comprising:

- one or more alkaline agents, and
- one or more oxidation dyes and/or one or more direct dyes;

c) the composition (c) comprising one or more oxidizing agents, and then
in applying the composition thus obtained to said wet or dry keratin fibres,
the composition obtained after mixing being such that the amount of fatty substances is greater than 20% by weight.

**2.** Process according to the preceding claim, **characterized in that** the aqueous cosmetic composition (A) comprises more than 5% by weight of water, preferably more than 10%, more particularly still more than 20% by weight, of water.

**3.** Process according to any one of the preceding claims, **characterized in that** the fatty substance(s) is (are) chosen from compounds that are liquid or pasty at room temperature and at atmospheric pressure.

**4.** Process according to any one of the preceding claims, **characterized in that** the concentration of fatty substances is from 25% to 80% by weight and preferably from 25% to 65% by weight relative to the total weight of composition (A).

**5.** Process according to any one of the preceding claims, **characterized in that** the aqueous composition (A) comprises one or more surfactants chosen from nonionic surfactants, in particular monooxyalkylenated or polyoxyalkylenated, and monoglycerolated or polyglycerolated nonionic surfactants.

**6.** Process according to any one of the preceding claims, **characterized in that** the alkaline agent(s) are chosen from organic amines and salts thereof, inorganic bases and ammonium salts.

**7.** Process according to the preceding claim, **characterized in that** the alkaline agent is monoethanolamine.

**8.** Process according to any one of the preceding claims, **characterized in that** composition (B) has a content of alkaline agents ranging from 0.01% to 30% by weight and preferably from 0.1% to 20% by weight relative to the weight of the said composition.

**9.** Process according to any one of the preceding claims, **characterized in that** the aqueous composition (C) comprises one or more oxidizing agents chosen from hydrogen peroxide, urea peroxide and alkali metal bromates or ferricyanides.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- CA 2573567 A1
- US 5817155 A
- GB 1026978 A
- GB 1153196 A
- FR 2801308
- DE 2359399
- JP 63169571 A
- JP 5063124 A
- EP 0770375 A
- WO 9615765 A
- DE 3843892
- DE 4133957
- WO 9408969 A
- WO 9408970 A
- FR 2733749 A
- DE 19543988
- FR 2886136 A
- WO 9515144 A
- WO 9501772 A
- EP 714954 A
- FR 2189006
- FR 2285851
- FR 2140205
- EP 1378544 A
- EP 1674073 A
- EP 1637566 A
- EP 1619221 A
- EP 1634926 A
- EP 1619220 A
- EP 1672033 A
- EP 1671954 A
- EP 1671955 A
- EP 1679312 A
- EP 1671951 A
- EP 167952 A
- EP 167971 A
- WO 06063866 A
- WO 06063867 A
- WO 06063868 A
- WO 06063869 A
- EP 1408919 A
- EP 1377264 A
- EP 1377262 A
- EP 1377261 A
- EP 1377263 A
- EP 1399425 A
- EP 1399117 A
- EP 1416909 A
- EP 1399116 A
- EP 1671560 A
- EP 1006153 A
- EP 1433472 A
- EP 1433474 A
- EP 1433471 A
- EP 1433473 A
- EP 6291333 A